# EUROPEAN PATENT SPECIFICATION

(11) **EP 4 356 887 B1**
(45) Date of publication and mention of the grant of the patent: **11.03.2026**
(21) Application number: 23195097.3
(22) Date of filing: 04.09.2023
(51) Int. Cl.: A61F 13/15, B65H 39/00

(54) **METHOD OF PREDICTIVE DETECTION OF DEFECTS IN ABSORBENT SANITARY ARTICLES**
VERFAHREN ZUR PRÄDIKTIVEN ERKENNUNG VON DEFEKTEN IN ABSORBIERENDEN HYGIENEARTIKELN
PROCÉDÉ DE DÉTECTION PRÉDICTIVE DE DÉFAUTS DANS DES ARTICLES SANITAIRES ABSORBANTS

(30) Priority: 18.10.2022 IT 202200021483
(43) Date of publication of application: 24.04.2024
(73) Proprietor: GDM S.p.A., 40133 Bologna (IT)
(72) Inventor: PIANTONI, Matteo, I-40133 Bologna (BO) (IT); ZAVALLONI, Alessandro, I-40133 Bologna (BO) (IT); CARUANA, Paolo, I-40133 Bologna (BO) (IT)
(74) Representative: Porta & Consulenti Associati S.p.A.

(56) References cited:
- WO-A1-2018/097171
- US-A1- 2004 030 432
- US-A1- 2013 304 246
- US-A1- 2019 258 233

## Description

The invention concerns a method of predictive detection of defects in absorbent sanitary articles and a production line of absorbent sanitary articles.

The absorbent sanitary articles are for example nappies for children, sanitary pads or tampons for women, pads for people suffering from incontinence and the like.

Typically, such articles are produced in a production line characterized by a high construction complexity, and at the same time the nappies themselves have a high structural complexity. For this reason, production can be affected by malfunctionings and defective articles.

EP 2 678 746 discloses a method of corrective intervention on the operation of a production line of absorbent sanitary articles in which it is envisaged to:
- detect at least one image of each article in output from the line;
- define by means of said image first parameters indicative of the positioning and/or of the assembly and/or of the shape of at least one respective component;
- detect a production defect in the event that at least one of said first parameters is outside a respective range of acceptability;
- identify second operating parameters of the line which are responsible for the definition of the first parameter resulted outside the respective range of acceptability;
- compare said operating parameters of the line with respective third reference parameters indicative of an optimal operation of the line;
- derive from the comparison a map of anomalous operating parameters;
- verify whether each combination of anomalous operating parameters is significant of a respective malfunctioning cause of the line, the respective cause falling within case studies of preset and defined malfunctioning causes;
- define, for each malfunctioning cause found, the corrective action to be taken to cancel the production defect.

The Applicant observes that EP 2 678 746 discloses a solution in which production defects are detected starting from an image processing of absorbent sanitary articles in output from the production line.

With respect to such a solution, the Applicant has perceived the need to improve the quality control of the production of sanitary absorbent articles, in particular in terms of reliability, costs and efficiency.

The Applicant has found that the aforesaid need can be met by an automated method of predictive detection based on a system for monitoring current values of operating parameters which are indicative of the operation of production devices of the line, on a processing of the current values of the monitored operating parameters which is adapted to detect any anomalous operating parameters and to derive therefrom a current malfunctioning cause of the line as well as on a further processing that allows to make a prediction of any defects, which could occur or be present in the absorbent sanitary articles, starting from said current malfunctioning cause identified.

The present invention therefore concerns, in a first aspect thereof, an automated computer-implemented method of predictive detection of defects in absorbent sanitary articles being processed along a production line and/or in finished absorbent sanitary articles in output from the production line.

It is provided to detect current values of operating parameters which are indicative of the current operation of at least one production device adapted to perform respective production operations on the absorbent sanitary articles being processed in the production line;
It is provided to perform a comparison between the current values of the detected operating parameters and respective predetermined reference values.

In the presence, among said operating parameters, of at least one anomalous operating parameter whose current value does not comply with the respective predetermined reference value, it is provided to identify, among a plurality of possible malfunctioning causes of the line, at least one current malfunctioning cause responsible for said at least one anomalous operating parameter, said at least one current malfunctioning cause being identified starting from said at least one anomalous operating parameter by means of decision algorithms which are based on at least one of the following information: known and/or self-learned data related to correlations between said at least one anomalous operating parameter and said plurality of possible malfunctioning causes of the line; current and/or historical information manually provided by an operator in association with said at least one anomalous operating parameter and/or with said plurality of possible malfunctioning causes of the line; known and/or self-learned logic rules.

By means of said decision algorithms, it is provided to make a prediction of the defects starting from said at least one current malfunctioning cause identified and based on at least one of the following information: known and/or self-learned data related to correlations between said at least one current malfunctioning cause identified and at least one known possible defect; current and/or historical information manually provided by an operator in association with said at least one current malfunctioning cause identified and/or said at least one known possible defect; known and/or self-learned logic rules.

The present invention concerns, in a second aspect thereof, a production line of absorbent sanitary articles.

The production line comprises production devices configured to perform respective production operations on the absorbent sanitary articles being processed along the production line.

The production line comprises sensors adapted to detect current values of operating parameters which are indicative of the current operation of at least one of the production devices.

The production line comprises a control unit comprising a memory and a processor configured to perform a comparison between the current values of the operating parameters detected by the sensors and respective predetermined reference values stored in said memory. In the presence, among said operating parameters, of at least one anomalous operating parameter whose current value does not comply with the respective predetermined reference value, said processor is preferably configured to identify, among a plurality of possible malfunctioning causes of the line, at least one current malfunctioning cause responsible for said at least one anomalous operating parameter. Preferably, the processor is configured to identify said at least one current malfunctioning cause starting from said at least one anomalous operating parameter by means of decision algorithms which are based on at least one of the following information stored in said memory: known and/or self-learned data related to correlations between said at least one anomalous operating parameter and said plurality of possible malfunctioning causes of the line; current and/or historical information manually provided by an operator in association with said at least one anomalous operating parameter and/or said possible malfunctioning causes of the line; known and/or self-learned logic rules.

Preferably, the processor is configured to make, by means of said decision algorithms, a prediction of any defects starting from said at least one current malfunctioning cause identified and based on at least one of the following information: known and/or self-learned data related to correlations between said at least one current malfunctioning cause identified and at least one known possible defect; current and/or historical information manually provided by an operator in association with said at least one current malfunctioning cause identified and/or said at least one known possible defect; known and/or self-learned logic rules.

The Applicant observes that, according to the invention, a predictive detection of defects that might occur or be already present in sanitary absorbent articles is performed starting from the detection of a current malfunctioning cause of the production line, which is identified based on parameters that are directly connected to the operation of the production devices of the articles (that is, based on parameters detected by sensors adapted to monitor the operation of the production devices of the articles). All this without having article data directly correlated to the absorbent sanitary articles and techniques that are based on a direct inspection of the articles, in particular on an image processing of the articles.

This advantageously makes it possible to predict, in line and in an automated way, also defects (such as, for example, those related to the tightness of a gluing or of a seal or to the degree of absorbency of an article) that - not being detectable starting from a visual inspection of the articles - are hardly detectable using traditional quality control systems, for example of the *machine vision* type (i.e. based on image processing techniques).

In particular, the invention makes it possible to predict such defects without requiring the use of advanced and expensive inspection devices or the execution of laboratory controls, outside the production line, on sample products. For example, this advantageously makes it possible to avoid carrying out quality controls adapted to test the tightness of a seal or of a gluing that are typically performed outside the production line on sample products by mechanically measuring, through conventional tensile tests, the maximum tensile force that the seal or the gluing is able to withstand until breakage, as a quality parameter.

The Applicant observes that, by providing a method that operates upstream of any detection of defects performed by direct inspection of the same articles, the invention advantageously allows to have real-time information on malfunctioning causes of the line that could generate defects on the articles and, therefore, promptly signal the presence of possible defective articles and/or immediately intervene on the production line in order to prevent the production of defective articles.

In addition, the Applicant observes that, according to the invention, a current malfunctioning cause of the line is identified based on parameters that are directly connected to the operation of the production devices, independently of any detection of defects performed on the absorbent sanitary articles.

This advantageously allows to identify the malfunctioning causes of the line in a reliable and precise way, considering that there are malfunctionings of the line that do not affect the quality of the articles produced. These are, for example, malfunctionings (such as wear or loss of energy efficiency of a machine) that do not necessarily generate defects on the absorbent sanitary articles produced by the line.

The Applicant further observes that given the high constructional complexity of the production line of absorbent sanitary articles and the high structural complexity of the same articles, an anomalous operating parameter could be generated by different possible malfunctioning causes of the line. Tracing back, among a plurality of possible malfunctioning causes, to the malfunctioning cause that is currently responsible for the anomalous operating parameter can be a non-immediate operation and require the intervention of experienced operators, provided with a high knowledge of the operation of the line, of the most frequent problems that can be found in the production lines of absorbent sanitary articles and with a good historical memory of malfunctioning causes identified in the past in association with predetermined values of anomalous operating parameters. Consider, for example, the case of identifying an anomalous operating parameter related to an irregular vibration of a device. This anomaly could be generated by different malfunctioning causes of the line such as, for example, the wear or breakage of a piece of the device itself, the malfunctioning of upstream devices that have caused the presence in the article of a seal or of a material with a thickness greater or smaller than expected, the blockage or the misalignment of a piece of the device itself and the like.

According to the invention, all this is overcome thanks to the decision algorithms that allow - without the intervention of experienced operators - to automatically trace, among a plurality of possible malfunctioning causes, the one which has currently caused the anomalous operating parameter and to predict a defect starting from the malfunctioning cause thus identified.

Overall, the objectives set forth above of improving the quality control of the production of absorbent sanitary articles, in particular in terms of reliability, costs and efficiency are achieved.

By "absorbent sanitary article being processed" or "article being processed" is meant a semi-processed absorbent sanitary article at any step of the relative production process within a production line, including individual materials and individual elements forming the absorbent sanitary article.

By the term "finished absorbent sanitary article" or "finished article" is meant a finished absorbent sanitary article obtained at the end of the production process.

By "absorbent sanitary article" or "article" is meant a finished absorbent sanitary article or an absorbent sanitary article being processed.

By "malfunctioning" referred to a production line is meant a malfunctioning that may concern any operating device in the production line, including both a production device that is configured to perform a respective production operation on the absorbent sanitary articles being processed along the production line and an accessory device with respect to production, such as a device adapted to maintain adequate boundary conditions within the plant in which the production line is housed, such as for example a device adapted to maintain a certain level of temperature and/or humidity within the plant.

Such malfunctioning could generate a defect in the absorbent sanitary articles and/or cause a stop (preferably automatic) of the line or of the malfunctioning production device.

By "defect" is meant to indicate an imperfection that is present or may occur in an absorbent sanitary article with respect to a predefined standard.

By "advancement direction" referred to an article within a production line is meant to indicate a direction of movement of that article within that line. For example, in the case of an article moved by transport members (comprising, for example, a conveyor belt) it is meant to indicate a direction of movement of said transport members (in the example of said conveyor belt).

The present invention may have, in the aspects discussed above, at least one of the preferred characteristics disclosed below. These characteristics may therefore be present individually or in combination with each other, unless expressly stated otherwise.

The method of predictive detection is a computer-implemented method.

The predictive detection preferably comprises the prediction both of any defects that might occur (but are not yet present) in the absorbent sanitary articles being processed along the production line and/or in the finished absorbent sanitary articles in output from the line and defects that are already present in such articles.

Preferably, any defects predicted by means of said decision algorithms relate to at least one of: tightness of a seal, tightness of a gluing, distribution or quantity of material and degree of absorbency.

The defectiveness or not of a tightness of a seal or of a gluing can relate to the maximum stress (such as, for example, tensile force) that the seal is able to withstand until breakage.

The predetermined reference values can be represented by individual values (in this case the comparison is performed to verify that the current values of the operating parameters detected by the sensors are greater or lower than the respective reference values) or by ranges of values (in this case the comparison is performed to verify that the current values of the operating parameters detected by the sensors are inside or outside the respective ranges of reference values).

In one embodiment, one or more of the sensors are adapted to detect current values of operating parameters whose anomaly with respect to the respective predetermined reference values generates a stop (preferably automatic) of the line or of the relative production device. Preferably, the processor is configured to perform (i.e. trigger) said comparison between the current values of the detected operating parameters and the respective reference values continuously.

Preferably, the processor is configured to perform said comparison independently of any detection of defects performed directly on the absorbent sanitary articles being processed along the line and on the finished absorbent sanitary articles, in output from the line. That is, the processor is configured to perform said comparison continuously regardless of whether or not such detection of defects is performed directly on the sanitary absorbent articles.

In a preferred embodiment, the line comprises at least one inspection device configured to acquire article data directly correlated to the absorbent sanitary articles being processed along the production line and/or to the finished absorbent sanitary articles, in output from the line.

The article data can relate, for example, to the appearance, shape, weight, positioning, dimensions, contours of the articles being processed along the production line and/or to the finished absorbent sanitary articles, in output from the line.

Said at least one inspection device may, for example, be selected from: an image acquisition device, a weight sensor, a proximity sensor, a photoelectric sensor, a contour sensor, a displacement sensor, a position sensor and a quantity sensor.

Preferably, the processor is configured to perform a detection of any further defects on the absorbent sanitary articles being processed along the production line and/or on the finished absorbent sanitary articles in output from the line, by processing the article data acquired by said at least one inspection device.

This detection of further defects performed by processing the article data acquired by said at least one inspection device is distinct from the prediction of any defects and can be advantageously performed in addition thereto in order to increase the reliability of the quality control of the production process.

In a preferred embodiment, the line comprises an image acquisition device (e.g., a camera) configured to acquire images of the absorbent sanitary articles being processed along the production line and/or images of the finished absorbent sanitary articles, in output from the line.

Preferably, the processor is configured to perform a detection of any further defects on the absorbent sanitary articles being processed along the production line and/or on the finished absorbent sanitary articles in output from the line, by processing the images acquired by the image acquisition device.

This detection of further defects performed by processing the images acquired by the image acquisition device is distinct from the prediction of any defects and can be advantageously performed in addition thereto in order to increase the reliability of the quality control of the production process.

Preferably, the processor is configured to perform said comparison between the current values of the operating parameters detected by the sensors and the respective reference values continuously, independently of said detection of any further defects performed by processing the article data or, specifically, by processing said images. That is, the processor is configured to trigger said comparison regardless of whether or not a further defect is detected by processing the article data or, specifically, by processing said images. In particular, the detection of a further defect by processing the article data or, specifically, by processing said images is not necessary for the purpose of the activation of said comparison. However, this does not exclude that data related to further defects detected by processing the article data or, specifically, by processing said images may be used by said decision algorithms.

Preferably, the processor is configured to store in said memory said at least one current malfunctioning cause identified in association with said at least one anomalous operating parameter so as to enrich the self-learned data related to correlations between said at least one anomalous operating parameter and said plurality of possible malfunctioning causes of the line.

Preferably, the processor is configured to store in said memory any predicted defects so as to enrich the self-learned data related to said correlations between said at least one current malfunctioning cause identified and said at least one known possible defect.

Preferably, the processor is configured to define, by means of said decision algorithms, a corrective action to be taken to remedy said at least one current malfunctioning cause identified and said any predicted defects.

The corrective action to be taken may concern one or more of the production devices or one or more of accessory devices (with respect to production) associated with the production line, which are involved in the current malfunctioning cause identified.

For example, an accessory device could be a device adapted to maintain adequate boundary conditions within the plant in which the production line is housed, such as for example a device adapted to maintain a certain level of temperature and/or humidity within the plant.

Preferably, the line comprises actuators adapted to implement the corrective action to be taken.

Preferably, the processor is configured to automatically convert the corrective action to be taken into a control signal for the actuators.

Preferably the actuators are operatively associated with said control unit.

Preferably, the actuators are operatively associated with at least one of the production devices.

Preferably, the actuators are configured to intervene on the operation of at least one of the production devices based on the control signal coming from the processor.

Preferably, the actuators are operatively associated with one or more accessory devices with respect to the production of the articles, which are present within the production line.

Preferably, the actuators are configured to intervene on the operation of one or more of the accessory devices based on the control signal coming the processor.

Preferably, the production devices comprise at least one of:
- transport members adapted to support and move the absorbent sanitary articles being processed along the line;
- feeding devices adapted to feed elements of the absorbent sanitary articles being processed (for example in the form of strips) and to place them, at least partially mutually overlapping, on a supporting surface of the transport members;
- retaining members configured to retain in position the elements placed on the supporting surface of the transport members, preferably comprising suction devices active on retaining holes formed on the supporting surface of the transport members;
- at least one fixing device adapted to fix the elements of the absorbent sanitary articles being processed together (for example by sealing and/or gluing);
- at least one cutting device adapted to cut elements of the absorbent sanitary articles being processed and/or a continuous strip of the absorbent sanitary articles being processed joined together, resulting from the production process, into individual articles.

Preferably, the sensors comprise at least one of: temperature sensor; pressure sensor; vibration sensor (e.g., accelerometer); position sensor; weight sensor; quantity sensor; flow sensor (e.g., flow meter); vacuum level sensor; air jet status sensor; optical sensor; force sensor (e.g., load cells); speed sensor; torque sensor; current sensor.

Preferably, the operating parameters detected by the plurality of sensors comprise at least one of: temperature; pressure; vibration; position of objects; weight; quantity; vacuum level; status of air jets; impact energy level of the cutting device; level of cleanliness; presence or quantity of material at a predetermined position; feeding tension of the elements of the absorbent sanitary articles being processed; speed; torque (e.g. of a motor) and current absorbed (e.g. by a motor).

Preferably, said decision algorithms are implemented by means of artificial intelligence, expert system or preset and defined logics and correlations.

Preferably, said decision algorithms are assisted by suitable machine learning and/or statistical algorithms.

Preferably, in the case of several anomalous operating parameters, the processor is configured to identify said at least one current malfunctioning cause both starting from each individual anomalous operating parameter and starting from various possible combinations of said anomalous operating parameters.

Preferably, in the case of identifying several malfunctioning causes, the processor is configured to make the prediction of any defects both starting from each individual malfunctioning cause identified and starting from various possible combinations of malfunctioning causes identified.

In a preferred embodiment, it is provided to acquire article data directly correlated to the absorbent sanitary articles being processed along the production line and/or to the finished absorbent sanitary articles, in output from the line.

The article data can relate, for example, to the appearance, shape, weight, positioning, dimensions, contours of the articles being processed along the production line and/or to the finished absorbent sanitary articles, in output from the line.

Preferably, it is provided to detect any defects on the absorbent sanitary articles being processed along the production line and/or on the finished absorbent sanitary articles, in output from the line, by processing said article data.

Preferably, it is provided to perform said comparison continuously (for example according to a predefined cadence) independently of said detection of any defects performed by processing said article data.

In a preferred embodiment, it is provided to acquire images of the absorbent sanitary articles being processed along the production line and/or images of the finished absorbent sanitary articles, in output from the line.

Preferably, it is provided to detect any further defects on the absorbent sanitary articles being processed along the production line and/or on the finished absorbent sanitary articles, in output from the line, by processing said images.

Preferably, it is provided to perform said comparison continuously (for example according to a predefined cadence).

Preferably, it is provided to perform said comparison between the current values of the detected operating parameters and the respective predetermined reference values continuously (for example according to a predefined cadence), independently of said detection of any further defects performed by processing said images.

Preferably, it is provided to store said at least one current malfunctioning cause identified in association with said at least one anomalous operating parameter so as to enrich the self-learned data related to correlations between said at least one anomalous operating parameter and said plurality of possible malfunctioning causes of the line.

Preferably, it is provided to store any predicted defects so as to enrich the self-learned data related to said correlations between said at least one current malfunctioning cause identified and said at least one known possible defect.

Preferably, it is provided to define, by means of said decision algorithms, a corrective action to be taken to remedy said at least one current malfunctioning cause identified and said any predicted defects.

Generally, the detected current malfunctioning cause concerns one or more of the production devices or other accessory device (with respect to production) associated with the production line, which is different from the production device to which the anomalous operating parameter identified is associated.

The corrective action to be taken may concern one or more of the production devices and/or other accessory device associated with the production line, which are involved in the current malfunctioning cause identified.

Preferably, it is provided to implement the defined corrective action by intervening on one or more of the production devices or other accessory device.

Preferably, the article comprises a plurality of elements.

Preferably, said elements are at least partly strip-shaped.

The elements can be distinguishable into base elements and additional elements.

The base elements preferably comprise a first sheet of permeable material and a second sheet of impermeable material, which are intended to define, respectively, the inner face and the outer face of the article.

The first sheet and the second sheet are typically mutually overlapping and between them there is interposed a third main element constituted by an absorbent padding.

The additional elements, which may vary in number and shape, preferably comprise front and/or rear flaps, acquisition and distribution layers or reinforcement layers or other discrete elements typically envisaged in the absorbent sanitary articles between the first sheet and the second sheet.

Preferably, within the production line, the absorbent sanitary articles being processed can be moved along an advancement direction.

Preferably, the transport members are adapted to support and move the absorbent sanitary articles being processed along said advancement direction.

In one embodiment, the transport members comprise at least one conveyor belt.

Preferably, the conveyor belt can be moved along the advancement direction for the movement of the articles being processed within the production line.

The conveyor belt may have an at least partly flat and/or curved supporting surface.

The advancement direction may be at least partly straight and/or curved.

Further characteristics and advantages of the present invention will become clearer from the following detailed disclosure of a preferred embodiment thereof, made with reference to the appended drawings and provided by way of indicative and non-limiting example, in which:
- Figure 1 schematically shows a production line of absorbent sanitary articles according to an embodiment of the invention;
- Figure 2 represents, in plan, a schematic example of an absorbent sanitary article that can be made by the line of Figure 1.

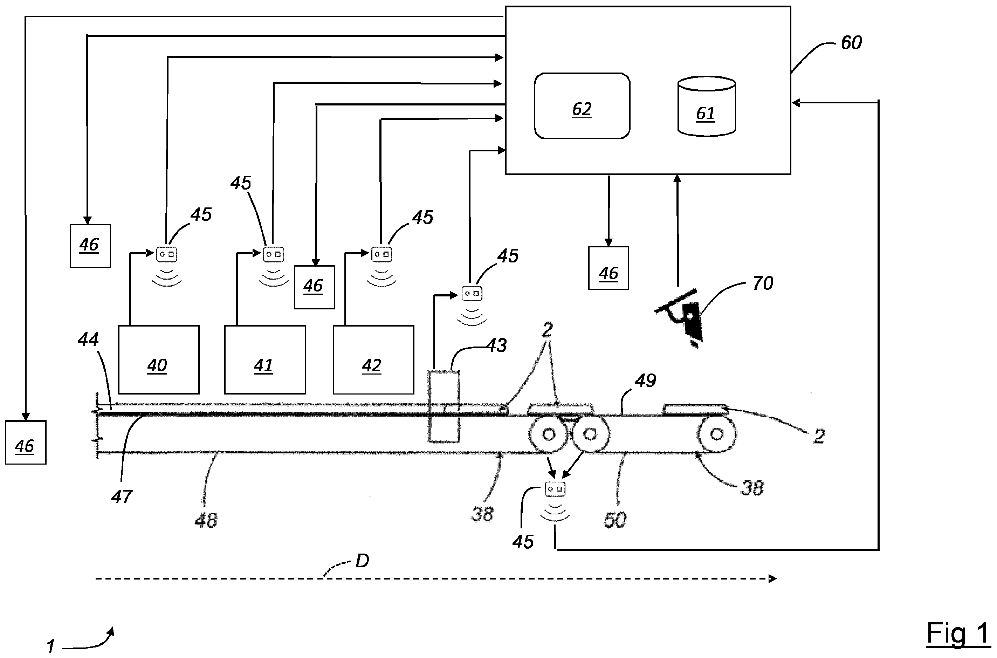

Figure 1 schematically shows a production line 1 of absorbent sanitary articles 2 according to an embodiment of the invention.

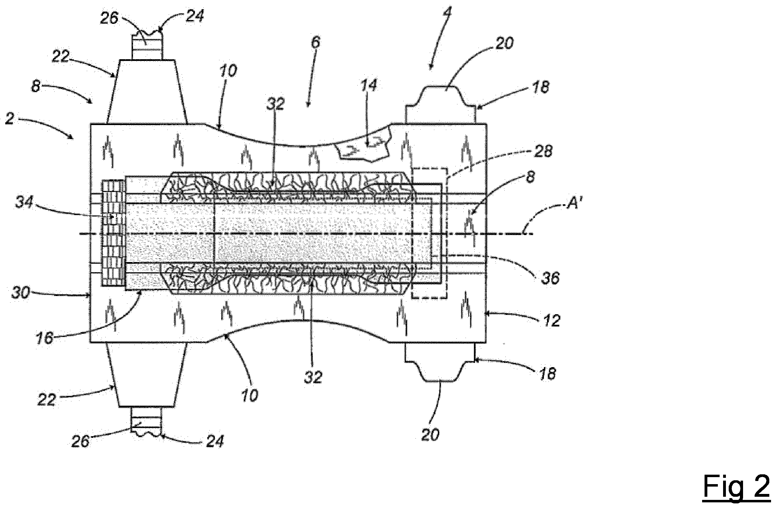

With reference to Figure 2, the absorbent sanitary articles 2 have a substantially rectangular shape. In the specific example the articles 2 are nappies for children, however, in the spirit of the present invention and according to variants immediately deducible from the context and from what is disclosed below, the articles 2 may be sanitary pads for women, pads for senile incontinence or the like.

The articles 2 comprise, in alignment according to a longitudinal axis thereof, indicated with A', a front portion 4, wearable on the front part of the body of the user, a rear portion 8, wearable on the rear part of the body of the user, and a central portion 6, arranged between the front part and the rear part and wearable between the legs of the user. At the central portion 6 there is provided a recess 10, or leg line, defined by two arcuate sections that are symmetrical with respect to the axis A".

The absorbent sanitary articles 2 are formed by a plurality of elements, distinguishable into base elements and additional elements. In particular, the base elements are assembled together in such a way as to define a base article 2, whose elements are arranged according to a preset and defined scheme. Similarly, the additional elements are applied on the base article during and/or after the definition thereof, according to a preset and defined scheme, so as to realize a finished article 2.

The base elements are constituted by a first sheet 12 of permeable material (non-woven fabric) and a second sheet 14 of impermeable material, which are intended to define, respectively, the inner face and the outer face of the article 2.

The sheets 12 and 14 are mutually overlapping and between them there is interposed a third main element constituted by an absorbent padding 16. The padding 16 is formed by cellulose fibre and superabsorbent polymer (SAP) granules dispersed therein.

The additional elements, which may vary in number and shape, are disclosed below with reference to the absorbent sanitary article 2 illustrated in Figure 2.

18 indicates two first shaped wings, each having a respective lobe 20, fixed to the inner face of the second impermeable sheet 14 and extending transversely to the axis A' from the front portion 4 of the article, and 22 indicates two shaped closing wings, parallel to the first wings 18, extending from the rear portion 8 of the article 2.

To each of the second closing wings 22 there is applied a third flap 24 provided with an adhesive strip 26, with a development parallel to the axis A', intended to adhere, in use, to a corresponding front strip 28 applied to the front portion 4 of the outer face of the second sheet 14. The wings 22 provided with the flaps 24 constitute, together with the front strip 28 means for closing the article 2.

The adhesive strip 26 can be replaced, according to a variant, with a Velcro^{®} strip.

To the first sheet 12 of permeable material there are sealed laterally two strips 30 of impermeable material for thickening and expanding the longitudinal edges thereof. The strips 30 are provided at their intermediate section with an elasticized portion 32.

A further additional component is constituted by an elastic band 34 applied, transversely to the axis A', to the inner face of the second sheet 14 at the rear portion 8 of the article 2.

At the inner face of the first sheet 12 of permeable material, in contact with the absorbent padding 16 and sealed along the contour of the latter, there is provided a sheet 36 of absorbent material, defined as "acquisition layer", having the function of making the absorbent power of the surface of the absorbent padding 16 uniform.

With reference to Figure 1, the production line 1 comprises a plurality of production devices 38, 40, 41, 42, 43 of known type adapted to perform respective production operations on the absorbent sanitary articles 2 being processed along the line 1.

Such operations may comprise, for example, support and movement; feeding of material constituting the base elements and the additional elements and/or the elements themselves; cutting the materials to make the individual elements or to shape the elements already applied; placing the base elements and the additional elements in partial or total mutual overlapping; fixing the various base and additional elements by gluing and/or sealing; cutting a continuous strip 44 of the absorbent sanitary articles 2 being processed joined together, resulting from the production process, into individual articles, and the like.

In the embodiment illustrated in Figure 1, the production devices comprise transport members 38 adapted to support and move, along an advancement direction D, the absorbent sanitary articles 2 being processed along the line 1.

In the illustrated embodiment, the advancement direction D is straight.

The transport members 38 may comprise a plurality of conveyor belts. In particular, in Figure 1 there are indicated a first conveyor belt 48 with a respective supporting surface 47 and a second conveyor belt 50 with a respective supporting surface 49.

In the illustrated embodiment, the production devices also comprise feeding devices (indicatively and schematically illustrated in the figure with a single block 40), adapted to feed material constituting the base elements and the additional elements of the articles 2 being processed and/or the elements themselves and to place them, at least partially mutually overlapping, on a supporting surface 47 of the transport members 38.

For example, in the embodiment illustrated in Figure 1, the supporting surfaces 47 and 49 are flat.

In the embodiment illustrated in Figure 1, the production devices also comprise retaining members (indicatively and schematically illustrated in the figure with a single block 41) configured to retain in position the elements/materials placed on the supporting surface 47 of the first conveyor belt 48 and to retain in position the finished articles 2 on the transport surface 49 of the second conveyor belt 50. For example, the retaining members 41 comprise suction devices (not illustrated) active on retaining holes (not illustrated) formed on at least part of the supporting surface 47 of the first conveyor belt 48 and on at least part of the transport surface 49 of the second conveyor belt 50.

In the embodiment illustrated in Figure 1, the production devices also comprise at least one fixing device (indicatively and schematically illustrated in the figure with a single block 42) adapted to fix the base and additional elements together by gluing and/or sealing.

Said at least one fixing device 42 may comprise an ultrasonic sealing device.

In an alternative embodiment, said at least one fixing device 42 may comprise a thermomechanical sealing device.

In addition or alternatively, said at least one fixing device 42 may comprise a gluing device comprising a glue dispensing device associated with a tank (not illustrated) for the glue. In one embodiment, said glue dispensing device may comprise inside it a small secondary glue tank adapted to draw the glue, by means of a suitable drawing mechanism, from an external primary glue tank.

In the embodiment illustrated in Figure 1, the production devices also comprise at least one cutting device (indicatively and schematically illustrated in the figure with a single block 43) adapted to perform the aforesaid cutting operations. In particular, Figure 1 depicts a device adapted to make cuts or transverse weakening lines (with respect to the advancement direction D) of the continuous strip 44 of absorbent sanitary articles being processed 2 in such a way as to be able to subsequently separate or make the absorbent sanitary articles 2 easily separable.

One or more of the production devices 40, 41, 42, 43 may be made by an individual device or by separate devices. Such an individual device or such separate devices may comprise one or more forming drums (not illustrated) which are rotatable about respective axes of rotation. In such a case, at least one between the first conveyor belt 48 and the second conveyor belt 50 may be wholly or partly replaced by transport surfaces of such forming drums.

The supporting surfaces 47 and 49 of the transport members 38 may thus be at least partly flat and/or curved. Furthermore the advancement direction D may be at least partly straight and/or curved.

The production line 1 also comprises sensors 45 distributed along the line 1. The sensors 45 are operatively associated with the production devices 40, 41, 42, 43, 38. In particular, the sensors 45 are configured to detect current values of operating parameters of the respective production devices 40, 41, 42, 43, 38.

The operating parameters of the respective production devices 40, 41, 42, 43, 38 are linked to the process of positioning, assembling and/or shaping the various elements constituting the article 2.

For example, the sensors 45 may comprise one or more temperature sensors, for example for measuring the temperature of the glue contained in the tank (not illustrated) associated with said at least one fixing device 42; one or more pressure sensors, for example adapted to measure a vacuum level created by the suction devices of the retaining members 41 and/or the pressure of the glue dispensed by said at least one fixing device 42; one or more vibration sensors such as, for example, an accelerometer adapted to measure, for example, the vibrations of a piece (such as, for example, the supporting surface 47 of the first conveyor belt 48) of a production device 38, 40, 41, 42, 43; one or more position sensors, for example adapted to measure the position of an object with respect to a reference or the mutual position of objects at one or more of the production devices 38, 40, 41, 42, 43. For example, the position sensors may be adapted to measure the position of a roller (not shown) of the feeding devices 40 with respect to an application point and/or the opening/closing of a shutter of the suction devices of the retaining members 41. The sensors 45 may further comprise one or more weight sensors (e.g. load cells), for example adapted to control the weight of the glue contained in the tank associated with said at least one fixing device 42 or the presence/absence/quantity of material at a predetermined position; one or more air jet status sensors, for example adapted to detect the status (active/inactive/intensity) of the air jets created by the suction devices of the retaining members 41 (for example by measuring the pressure of the air upstream by the suction devices of the retaining members 41); one or more optical sensors, for example adapted to detect the level of cleanliness at a production device 38, 40, 41, 42, 43; one or more force sensors (for example load cells), for example adapted to detect the feeding tension of the elements/materials of the absorbent sanitary articles fed by the feeding devices 40 and/or to verify whether compression members/rollers at the feeding devices 40 are operating correctly. Load cells can, for example, be used also to detect the impact energy level of said at least one cutting device 43. In addition or alternatively, the sensors 45 may comprise one or more sensors adapted to detect operating parameters of one or more motors present at least at one production device 38, 40, 41, 42, 43, such as the torque, the temperature and the absorbed current.

In a preferred embodiment illustrated in Figure 1, the production line 1 also comprises an image acquisition device 70 (e.g., a camera) configured to acquire images of the finished absorbent sanitary articles 2, in output from the line 1.

The image acquisition device 70 may be arranged facing the second conveyor belt 50, as illustrated in Figure 1, to detect at least one image of each finished article 2, i.e. already separated from the continuous strip 44 of articles 2 joined together, or, according to a variant not illustrated, the image acquisition device 70 may be arranged facing the first conveyor belt 48 to detect at least one image of each article 2 when it has yet to be separated from the continuous strip 44 of articles 2 joined together. In both cases, the image acquisition device 70 is arranged at the output of the line 1 to detect at least one image of each article 2 already defined relatively to the composition and assembly of the base and additional elements thereof.

The production line 1 also comprises a control unit 60 comprising a memory 61 and a processor 62.

The processor 62 is configured to implement a method according to an embodiment of the present invention.

The control unit is operatively connected (by means of a suitable wired and/or wireless connection) to the sensors 45 and to the image acquisition device 70.

In the preferred embodiment illustrated in Figure 1, the processor 62 is configured to detect any defects present in the finished absorbent sanitary articles 2, in output from the line. The detection of defects is performed by processing the images acquired by the image acquisition device 70 by means of suitable *machine vision* algorithms known in the art.

Typical defects that can be detected by said image processing may comprise: wrong alignment of the absorbent padding 16, wrong orientation of the front strip 28, wrong positioning of the closing wings 22 and wrong execution of the cut of the recess 10. These defects generally concern the positioning, the assembly and/or the shape of the various elements constituting the article 2 and are directly visible in the images acquired by the image acquisition device 70 and directly obtainable therefrom.

The processor 62 is further configured to perform continuously a comparison between the current values of the operating parameters detected by the sensors 45 and respective predetermined reference values stored in the memory 61.

When at least one of the detected current values does not comply with the respective predetermined reference value, the processor 62 labels the respective operating parameter as an anomalous operating parameter and is configured to identify, among a plurality of possible malfunctioning causes of the line 1, at least one current malfunctioning cause that is responsible for the anomalous operating parameter.

The malfunctioning cause may concern any operating device in the production line, including both a production device 38, 40, 41, 42, 43 and an accessory device (not illustrated) with respect to production, such as a device adapted to maintain adequate boundary conditions within the plant in which the production line 1 is housed, such as for example a device adapted to maintain a certain level of temperature and/or humidity within the plant.

The current malfunctioning cause identified concerns one or more of the production devices 38, 40, 41, 42, 43 and/or one or more of the accessory devices, which may be different from the production device 38, 40, 41, 42, 43 for which the anomalous operating parameter was detected.

In general, the anomalous operating parameter is not directly correlated to the current malfunctioning cause, which can be identified among a plurality of possible malfunctioning causes that could have generated such anomalous operating parameter.

The processor 62 is configured to identify said at least one current malfunctioning cause starting from said anomalous operating parameter by means of decision algorithms which are based on at least one of the following information stored in said memory: known and/or self-learned data related to correlations between said at least one anomalous operating parameter and said plurality of possible malfunctioning causes of the line; current and/or historical information manually provided by an operator in association with said at least one anomalous operating parameter and/or said possible malfunctioning causes of the line; known and/or self-learned logic rules.

According to the invention, the comparison between the current values of the operating parameters detected by the sensors 45 and respective reference values is not activated by the detection of defects performed by processing the images acquired by the image acquisition device 70. The processor is in fact configured to perform the comparison continuously, independently of said detection, that is to say regardless of whether or not a defect is detected by processing said images. However, this does not exclude that data related to defects detected by processing said images may be used by said decision algorithms.

The aforesaid decision algorithms can be implemented by means of techniques known in the art that make use of artificial intelligence, expert system or preset and defined logics as well as a modelling based on a study of the most frequent defects and problems that can be found in the production lines of absorbent sanitary articles.

In addition, the decision algorithms can be assisted by suitable statistical algorithms and suitable machine learning algorithms.

Preferably, in the case of several anomalous operating parameters, the processor 62 is configured to identify the current malfunctioning cause starting both from each individual anomalous operating parameter and from various possible combinations of said anomalous operating parameters.

Preferably, the processor 62 is configured to store in the memory 61 each current malfunctioning cause identified in association with the respective anomalous operating parameter(s) so as to enrich the self-learned data related to correlations between anomalous operating parameters and possible malfunctioning causes of the line.

According to the invention, the processor 62 is further configured to make, by means of the aforesaid decision algorithms, a prediction of defects that might occur or be already present in the absorbent sanitary articles being processed along the line 1 and/or in the finished absorbent sanitary articles in output from the line 1. Such prediction is perform starting from said at least one current malfunctioning cause identified and based on at least one of the following information stored in said memory: known and/or self-learned data related to correlations between said at least one current malfunctioning cause identified and at least one possible defect; current and/or historical information manually provided by an operator in association with said at least one current malfunctioning cause identified and/or said at least one possible defect; known and/or self-learned logic rules.

For example, this prediction can be performed with the aid of a database in which every possible malfunctioning cause is correlated to a related defect. This database can be preset and defined on the basis of a modelling based on a study of known and/or self-learned data related to the most frequent defects and problems that can be found in the production lines of absorbent sanitary articles.

The processor 62 is also configured to store in the memory 61 any defects thus predicted so as to enrich the self-learned data related to said correlations between malfunctioning causes and possible defects.

The Applicant observes that the invention advantageously allows to predict, in line and in an automated way, also defects that - not being directly detectable starting from a visual inspection of the articles- are hardly detectable starting from a processing of the images acquired by the image acquisition device 70.

The invention therefore allows, alternatively or preferably in addition to the detection of defects performed starting from a processing of the images acquired by the image acquisition device 70, to perform a predictive detection of defects and thus to control the production of articles 2 in an extremely reliable and precise manner.

Examples of non-visible defects that can be predicted by means of said decision algorithms can relate to: tightness of a seal, tightness of a gluing, distribution or quantity of material in the absorbent padding 16 and degree of absorbency of the absorbent padding 16.

Once said at least one current malfunctioning cause has been identified, the processor 62 is configured to perform at least one of the following actions: generate an alarm signal; generate a warning signal that the line 1 is making potentially defective articles; generate a warning signal on the need to discard the articles currently being produced; automatically stop the production line 1 or at least one of the production devices 38, 40, 41, 42, 43; define, by means of said decision algorithms, a corrective action to be taken to remedy said at least one current malfunctioning cause identified or said any predicted defects; signal the corrective action to be taken; indicate (for example by means of a display, not illustrated) a sequence of step-by-step instructions to guide an operator in implementing the corrective action.

The corrective action may concern one or more of the production devices 38, 40, 41, 42, 43 and/or one or more of the aforesaid accessory devices, which are involved in the current malfunctioning cause identified.

In the embodiment illustrated in Figure 1, the production line 1 comprises actuators 46 operatively associated with one or more of the production devices 38, 40, 41, 42, 43 and/or with one or more of said accessory devices. The actuators 46 are furthermore operatively connected (by means of a suitable wired and/or wireless connection) to said control unit 60.

The actuators 46 may, for example, be adapted to regulate the passage of the air at the suction devices of the retaining members 41 and may comprise shutters whose opening/closing level can be controlled automatically.

Additionally or alternatively, the actuators 46 may comprise, for example, adjustable valves and/or pressure regulators and/or temperature regulators.

The control unit is also adapted to perform a control action by sending, by the processor 62, appropriate control signals to the actuators 46 so that they implement the respective corrective action to be taken. The corrective actions are therefore automatically converted into control signals for the actuators 46 which act on the production devices 38, 40, 41, 42, 43 and/or on said accessory devices in order to remedy the malfunctioning identified, bring the anomalous operating parameters back in compliance with the respective reference values and avoid the production of defective articles.

With an appropriate feedback system, the processor 62 will be able to check that the corrective actions taken are effectively effective in bringing the anomalous operating parameters back in compliance with the respective reference values.

Consider, for example, the situation of a glue dispensing device in one of the fixing devices 42 which is provided in its inside with a small secondary glue tank adapted to draw the glue, by means of a suitable drawing mechanism, from an external primary glue tank. In the event that a temperature sensor acting as a sensor 45 associated with the secondary glue tank detects an anomalous temperature value, the processor 62- by means of the aforesaid decision algorithms- can identify, as possible malfunctioning causes a breakage or malfunctioning of the heating device (for example a coil) associated with the secondary glue tank or the presence of an inadequate quantity of glue in the secondary glue tank.

In the event that the processor 62 identifies - by means of the aforesaid decision algorithms - as a malfunctioning cause currently responsible for the anomalous temperature value the presence of an inadequate quantity of glue in the secondary glue tank, the processor 62 can for example predict that the articles could have gluings with non-optimal but nevertheless acceptable tightness (in this case, for example, the articles could be maintained at least for a production with medium-low quality standards).

Instead, in the event that the processor 62 identifies - by means of the aforesaid decision algorithms, as a malfunctioning cause currently responsible for the anomalous temperature value - a malfunctioning of the heating device, the processor 62 can for example predict that the articles could have gluings with unacceptable tightness (in this case, for example, the articles would be discarded also for a production with medium-low quality standards).

Furthermore, in the event that a frequency meter acting as a sensor 45 associated with an ultrasonic fixing device 42 detects an anomalous working frequency, the processor 62- by means of the aforesaid decision algorithms- can identify, as possible malfunctioning causes, a problem of misalignment between two sealing heads (sonotrode and anvil) of the fixing device 42, a problem of wear, breakage or malfunctioning of the sonotrode or of the fixing device 42 in general or a wrong setting of one of them.

In the event that the processor 62 identifies - by means of the aforesaid decision algorithms, as a malfunctioning cause currently responsible for the anomalous working frequency - a problem of misalignment between the two sealing heads (sonotrode and anvil), the processor can for example predict that the articles could have seals with non-optimal but nevertheless acceptable tightness (in this case, for example, the articles could be maintained at least for a production with medium-low quality standards).

Instead, in the event that the processor 62 identifies - by means of the aforesaid decision algorithms, as a malfunctioning cause currently responsible for the anomalous working frequency - a malfunctioning of the sonotrode, the processor could for example predict that the articles could have seals with unacceptable tightness (in this case, for example, the articles would be discarded also for a production with medium-low quality standards).

By way of further example, consider the case where a weight sensor (e.g. a load cell) acting as a sensor 45 associated with a feeding device 40 of the material constituting the padding 16 (e.g. cellulose fibre with super-absorbent polymer granules) detects an inadequate weight of such material placed on the transport surface 47 of the first conveyor belt 48. The processor 62- by means of the aforesaid decision algorithms- can identify, as possible malfunctioning causes, an exhaustion of such material in the feeding tank or a malfunctioning of the feeding device 40 which causes a dispensing flow of such material not compliant with the standard.

In the event that the processor 62 identifies - by means of the aforesaid decision algorithms, as a malfunctioning cause currently responsible for the inadequate weight - a malfunctioning of the feeding device 40, the processor can for example predict that the articles could have a padding 16 that is not optimal but nevertheless acceptable (in this case, for example, the articles could be maintained at least for a production with medium-low quality standards).

Instead, in the event that the processor 62 identifies - by means of the aforesaid decision algorithms - as a malfunctioning cause currently responsible for the inadequate weight an exhaustion of the material, the processor could for example predict that the articles could have an unacceptable padding 16 (in this case, for example, the articles would be discarded also for a production with medium-low quality standards).

Thanks to the invention, therefore, once the malfunctioning cause currently responsible for the anomalous operating parameter has been identified, it is possible to predict the presence or not of a defect in the absorbent sanitary articles and the extent of this defect (so as to determine, for example, whether the articles are to be discarded tout court or only for productions with high quality standards).

In addition, once the current malfunctioning cause is identified, the processor 62 is able to generate an alarm signal and define a corrective action to be taken to remedy the identified current malfunctioning cause so as to prevent or interrupt the production of defective articles.

As will be clear from the present disclosure, the advantages linked to the invention in its various aspects are manifold.

Thanks to the invention, a current malfunctioning cause of the line is identified on the basis of parameters that are directly connected to the operation of the production devices, independently of a detection of defects performed by a processing of the images acquired by the image acquisition device 70.

This advantageously allows to identify the malfunctioning causes of the line in a reliable and precise way, considering that there are malfunctionings of the line that, not affecting the quality of the articles produced or being hardly identifiable starting from a visual inspection of the articles produced by the line, cannot be identified starting from a detection of defects performed by processing the images acquired by the image acquisition device 70.

The Applicant also observes that, by providing a control method that operates upstream of any detection of defects performed directly on the same articles, the invention advantageously allows to have real-time information on malfunctioning causes that could generate production defects on the articles and, therefore, promptly signal the presence of possible defective articles and/or intervene immediately on the production line in order to prevent the production of defective articles.

In addition, the Applicant observes that there are defects (such as, for example, those related to the tightness of a gluing or of a seal or to the degree of absorbency of the absorbent padding) that - not being visible - are not identifiable by a processing of the images acquired by the image acquisition device 70. The latter defects, in order to be identified, would require advanced and expensive inspection devices or controls performed in the laboratory, outside the production line, on sample products.

The invention, by performing a predictive evaluation of defective articles by means of a solution that operates without *machine vision* techniques (i.e. image processing techniques), advantageously allows to predict, in the line and in an automated way, also non-visible defects, without requiring the use of advanced and expensive inspection devices or controls performed in the laboratory, outside the production line, on sample products.

Moreover, the invention advantageously allows to automatically identify, among a plurality of possible malfunctioning causes, the malfunctioning cause of the line that is currently responsible for one or more anomalous operating parameters without requiring the intervention of experienced operators.

## Claims

1. Automated computer-implemented method of predictive detection of defects in absorbent sanitary articles (2) being processed along a production line (1) and/or in finished absorbent sanitary articles in output from the production line (1), comprising the following steps:
- detecting current values of operating parameters which are indicative of the current operation of at least one production device (38, 40, 41, 42, 43) adapted to perform respective production operations on the absorbent sanitary articles (2) being processed along the production line (1);
- performing a comparison between the current values of the detected operating parameters and respective predetermined reference values;
- in the presence, among said operating parameters, of at least one anomalous operating parameter whose current value does not comply with the respective predetermined reference values, identifying, from among a plurality of possible malfunctioning causes of the line (1), at least one current malfunctioning cause responsible for said at least one anomalous operating parameter;
wherein said at least one current malfunctioning cause is identified starting from said at least one anomalous operating parameter by means of decision algorithms based on at least one of the following information: known and/or self-learned data related to correlations between said at least one anomalous operating parameter and said plurality of possible malfunctioning causes of the line; current and/or historical information manually provided by an operator in association with said at least one anomalous operating parameter and/or said plurality of possible malfunctioning causes of the line; known and/or self-learned logical rules; and
wherein, by means of said decision algorithms, a prediction of the defects is made starting from said at least one current malfunctioning cause identified and based on at least one of the following information: known and/or self-learned data related to correlations between said at least one current malfunctioning cause identified and at least one known possible defect; current and/or historical information manually provided by an operator in association with said at least one current malfunctioning cause identified and/or with said at least one known possible defect; known and/or self-learned logical rules.

2. Method according to claim 1, further comprising a step of storing said at least one current malfunctioning cause identified in association with said at least one anomalous operating parameter so as to enrich the self-learned data related to correlations between said at least one anomalous operating parameter and said plurality of possible malfunctioning causes of the line (1).

3. Method according to any one of the preceding claims, comprising a step of storing the predicted defects so as to enrich the self-learned data related to said correlations between said at least one current malfunctioning cause identified and said at least one known possible defect.

4. Method according to any one of the preceding claims, comprising a step of defining, by means of said decision algorithms, a corrective action to be taken to remedy said at least one current malfunctioning cause identified and said predicted defects; the corrective action to be taken relating to one or more of the production devices (38, 40, 41, 42, 43) and/or to one or more of accessory devices of the production line (1), which are involved in said current malfunctioning cause identified.

5. Method according to claim 4, further comprising a step of automatically converting the corrective action to be taken into a control signal for one or more actuators (46) which are operatively associated with said one or more production devices (38, 40, 41, 42, 43) and/or with said one or more accessory devices which are involved in the current malfunctioning cause identified.

6. Method according to any one of the preceding claims, comprising a step of acquiring the images of the absorbent sanitary articles (2) being processed along the production line (1) and/or of the absorbent sanitary articles (2) in output from the production line (1).

7. Method according to claim 6, comprising a step of detecting further defects by processing said acquired images, in addition to said prediction of the defects.

8. Method according to any one of the preceding claims, wherein the detected operating parameters comprise at least one of: temperature; pressure; vibration; position of objects; weight; quantity; vacuum level; status of air jets; impact energy level of a cutting device (43) of the line (1); level of cleanliness; presence or quantity of material at a predetermined position; feeding tension of elements of the absorbent sanitary articles (2) being processed; speed; torque; current.

9. Method according to any one of the preceding claims, wherein the defects predicted by means of said decision algorithms relate to at least one of: tightness of a seal, tightness of a gluing, distribution or quantity of material and degree of absorbency.

10. Method according to any one of the preceding claims, also comprising a step of acquiring article data directly related to the absorbent sanitary articles being processed along the production line and/or to the finished absorbent sanitary articles, in output from the line.

11. Method according to claim 10, wherein the article data relate to at least one of: appearance, shape, weight, positioning, dimensions and contours of the articles being processed along the production line and/or to the finished absorbent sanitary articles, in output from the line.

12. Method according to claim 10 or 11, comprising a step of detecting any further defects by processing said article data, in addition to said prediction of any defects.

13. Production line (1) of absorbent sanitary articles (12) comprising:
- production devices (38, 40, 41, 42, 43) configured to perform respective production operations on the absorbent sanitary articles (2) being processed along the production line (1);
- sensors (45) adapted to detect current values of operating parameters which are indicative of the current operation of at least one of the production devices (38, 40, 41, 42, 43);
- a control unit (60) comprising a memory (61) and a processor (62) configured to carry out the steps of the method according to any of claims 1 to 12.

14. Production line (1) according to claim 13, wherein the production devices (38, 40, 41, 42, 43) comprise at least one of:
- transport members (38) adapted to support and move the absorbent sanitary articles (2) being processed along the line (1);
- feeding devices (40) adapted to feed elements of the absorbent sanitary articles (2) being processed and to place them, at least partially mutually overlapping, on a supporting surface (47) of the transport members (38);
- retaining members (41) configured to retain in position the elements placed on the supporting surface (47) of the transport members (38), preferably comprising suction devices active on retaining holes formed on the supporting surface (47) of the transport members (38);
- at least one fixing device (42) adapted to fix the elements of the absorbent sanitary articles (2) being processed together;
- at least one cutting device (43) adapted to cut elements of the absorbent sanitary articles (2) being processed and/or a continuous strip (44) of the absorbent sanitary articles (2) being processed joined together, resulting from the production process, into individual articles (2);

15. Production line (1) according to any one of claims 13 and 14, wherein the sensors (45) comprise at least one of: temperature sensor; pressure sensor; vibration sensor; position sensor; weight sensor; quantity sensor; flow sensor; vacuum level sensor; air jet status sensor; optical sensor; tension sensor; speed sensor; torque sensor; current sensor.

## Patentansprüche

1. Automatisiertes, computerimplementiertes Verfahren zur prädiktiven Erkennung von Defekten in absorbierenden Hygieneartikeln (2), die entlang einer Produktionslinie (1) verarbeitet werden, und/oder in fertigen absorbierenden Hygieneartikeln im Ausgang aus der Produktionslinie (1), umfassend die folgenden Schritte:
- Erkennen von aktuellen Werten von Betriebsparametern, die Aufschluss über den aktuellen Betrieb von mindestens einer Produktionsvorrichtung (38, 40, 41, 42, 43) geben, die dazu geeignet ist, jeweilige Produktionsvorgänge an den absorbierenden Hygieneartikeln (2), die entlang der Produktionslinie (1) verarbeitet werden, durchzuführen;
- Durchführen eines Vergleichs zwischen den aktuellen Werten der erkannten Betriebsparameter und jeweiligen vorbestimmten Referenzwerten;
- wenn unter diesen Betriebsparametern mindestens ein anormaler Betriebsparameter enthalten ist, dessen aktueller Wert nicht mit dem jeweiligen vorbestimmten Referenzwert übereinstimmt, Identifizieren von mindestens einer aktuellen Störungsursache, die für den mindestens einen anormalen Betriebsparameter verantwortlich ist, aus einer Vielzahl von möglichen Störungsursachen der Linie (1),
wobei die mindestens eine aktuelle Störungsursache ausgehend von dem mindestens einen anormalen Betriebsparameter mittels Entscheidungsalgorithmen basierend auf mindestens einer der folgenden Informationen identifiziert wird: bekannten und/oder selbst erlernten Daten, die sich auf Korrelationen zwischen dem mindestens einen Betriebsparameter und der Vielzahl von möglichen Störungsursachen der Linie beziehen; aktuellen und/oder historischen Informationen, die manuell von einem Bediener in Verknüpfung mit dem mindestens einen anormalen Betriebsparameter und/oder der Vielzahl von möglichen Störungsursachen der Linie bereitgestellt werden; unbekannten und/oder selbst erlernten logischen Regeln, und
wobei mittels der Entscheidungsalgorithmen eine Vorhersage der Defekte ausgehend von der mindestens einen identifizierten aktuellen Störungsursache und basierend auf mindestens einer der folgenden Informationen erfolgt: bekannten und/oder selbst erlernten Daten, die sich auf Korrelationen zwischen der mindestens einen identifizierten aktuellen Störungsursache und dem mindestens einen bekannten möglichen Defekt beziehen; aktuellen und/oder historischen Informationen, die manuell von einem Bediener in Verknüpfung mit der mindestens einen identifizierten aktuellen Störungsursache und/oder mit dem mindestens einen bekannten möglichen Defekt bereitgestellt werden; unbekannten und/oder selbst erlernten logischen Regeln.

2. Verfahren nach Anspruch 1, ferner umfassend einen Schritt zum Speichern dieser mindestens einen identifizierten aktuellen Störungsursache in Verknüpfung mit dem mindestens einen anormalen Betriebsparameter, sodass die selbst erlernten Daten, die sich auf Korrelationen zwischen dem mindestens einen anormalen Betriebsparameter und die Vielzahl von möglichen Störungsursachen der Linie (1) beziehen, angereichert werden.

3. Verfahren nach einem der vorhergehenden Ansprüche, umfassend einen Schritt zum Speichern der vorhergesagten Defekte, sodass die selbst erlernten Daten, die sich auf die Korrelationen zwischen der mindestens einen identifizierten aktuellen Störungsursache und dem mindestens einen bekannten möglichen Defekt beziehen, angereichert werden.

4. Verfahren nach einem der vorhergehenden Ansprüche, umfassend einen Schritt zum Definieren einer zur Behebung der mindestens einen identifizierten aktuellen Störungsursache und der vorhergesagten Fehler zu ergreifenden Korrekturmaßnahme mittels der Entscheidungsalgorithmen, wobei sich die zu ergreifende Korrekturmaßnahme auf eine oder mehrere der Produktionsvorrichtungen (38, 40, 41, 42, 43) und/oder auf eine oder mehrere Zubehörvorrichtungen der Produktionslinie (1), die in die identifizierte aktuelle Störungsursache involviert sind, bezieht.

5. Verfahren nach Anspruch 4, ferner umfassend einen Schritt zum automatischen Umwandeln der zu ergreifenden Korrekturmaßnahme in ein Steuersignal für einen oder mehrere Aktoren (46), die betriebswirksam mit der einen oder den mehreren Produktionsvorrichtungen (38, 40, 41, 42, 43) und/oder mit der einen oder den mehreren Zubehörvorrichtungen, die in die identifizierte aktuelle Störungsursache involviert sind, verknüpft sind.

6. Verfahren nach einem der vorhergehenden Ansprüche, umfassend einen Schritt zum Erfassen der Bilder der absorbierenden Hygieneartikel (2), die entlang der Produktionslinie (1) verarbeitet werden, und/oder der absorbierenden Hygieneartikel (2) im Ausgang aus der Produktionslinie (1).

7. Verfahren nach Anspruch 6, umfassend einen Schritt zum Erkennen weiterer Defekte durch Verarbeiten der erfassten Bilder zusätzlich zur Vorhersage der Defekte.

8. Verfahren nach einem der vorhergehenden Ansprüche, wobei die erkannten Betriebsparameter mindestens einen der folgenden Parameter umfassen: Temperatur, Druck, Vibration, Position von Gegenständen, Gewicht, Menge, Vakuumniveau, Zustand von Luftdüsen, Aufprallenergieniveau einer Schneidvorrichtung (43) der Linie (1), Sauberkeitsgrad, Vorhandensein oder Menge von Material an einer vorbestimmten Position, Vorschubspannung von Elementen der verarbeiteten absorbierenden Hygieneartikel (2), Geschwindigkeit, Drehmoment, Stromstärke.

9. Verfahren nach einem der vorhergehenden Ansprüche, wobei sich die mittels der Entscheidungsalgorithmen vorhergesagten Defekte auf mindestens einen der folgenden Punkte beziehen: Dichtheit einer Dichtung, Dichtheit einer Verklebung, Verteilung oder Menge von Material und Grad der Saugfähigkeit.

10. Verfahren nach einem der vorhergehenden Ansprüche, zudem umfassend einen Schritt zum Erfassen von Artikeldaten, die sich direkt auf die entlang der Produktionslinie verarbeiteten absorbierenden Hygieneartikel und/oder die fertigen absorbierenden Hygieneartikel im Ausgang aus der Linie beziehen.

11. Verfahren nach Anspruch 10, wobei sich die Artikeldaten mindestens auf eines der folgenden Merkmale beziehen: Aussehen, Form, Gewicht, Positionierung, Abmessungen und Konturen der entlang der Produktionslinie verarbeiteten Artikel und/oder der fertigen absorbierenden Hygieneartikel im Ausgang aus der Linie.

12. Verfahren nach Anspruch 10 oder 11, umfassend einen Schritt zum Erkennen irgendwelcher weiterer Defekte durch Verarbeiten der Artikeldaten zusätzlich zur Vorhersage von Defekten.

13. Produktionslinie (1) für absorbierende Hygieneartikel (12), umfassend:
- Produktionsvorrichtungen (38, 40, 41, 42, 43), die ausgelegt sind, um jeweilige Produktionsvorgänge an den entlang der Produktionslinie (1) verarbeiteten absorbierenden Hygieneartikeln (2) durchzuführen;
- Sensoren (45), die geeignet sind, um aktuelle Werte von Betriebsparametern zu erkennen, die Aufschluss über den aktuellen Betrieb von mindestens einer der Produktionsvorrichtungen (38, 40, 41, 42, 43) geben;
- eine Steuereinheit (60), umfassend einen Speicher (61) und einen Prozessor (62), der ausgelegt ist, um die Schritte des Verfahrens nach einem der Ansprüche 1 bis 12 auszuführen.

14. Produktionslinie (1) nach Anspruch 13, wobei die Produktionsvorrichtungen (38, 40, 41, 42, 43) mindestens eines der folgenden Elemente umfassen:
- Transportelemente (38), die geeignet sind, um die entlang der Linie (1) verarbeiteten absorbierenden Hygieneartikel (2) zu stützen und zu bewegen;
- Zuführungsvorrichtungen (40), die geeignet sind, um Elemente der verarbeiteten absorbierenden Hygieneartikel (2) zuzuführen und diese zumindest teilweise überlappend auf einer Auflageoberfläche (47) der Transportelemente (38) zu platzieren;
- Halteelemente (41), die ausgelegt sind, um die auf der Auflageoberfläche (47) der Transportelemente (38) platzierten Elemente in Position zu halten, vorzugsweise umfassend Saugvorrichtungen, die auf auf der Auflageoberfläche (47) der Transportelemente (38) ausgebildeten Haltelöchern aktiv sind;
- mindestens eine Befestigungsvorrichtung (42), die geeignet ist, um die Elemente der verarbeiteten absorbierenden Hygieneartikel (2) aneinander zu befestigen;
- mindestens eine Schneidvorrichtung (43), die geeignet ist, um Elemente der verarbeiteten absorbierenden Hygieneartikel (2) und/oder einen sich aus dem Produktionsprozess ergebenden durchgehenden Streifen (44) der zusammengefügten verarbeiteten absorbierenden Hygieneartikel (2) in einzelne Artikel (2) zu schneiden;

15. Produktionslinie (1) nach einem der Ansprüche 13 und 14, wobei die Sensoren (45) mindestens einen der folgenden Sensoren umfassen: Temperatursensor, Drucksensor, Vibrationssensor, Positionssensor, Gewichtssensor, Mengensensor, Durchflusssensor, Vakuumniveausensor, Luftdüsenzustandssensor, optischer Sensor, Spannungssensor, Geschwindigkeitssensor, Drehmomentsensor, Stromstärkensensor.

## Revendications

1. Procédé automatisé mis en œuvre par ordinateur pour la détection prédictive de défauts dans des articles sanitaires absorbants (2) en cours de traitement le long d'une ligne de production (1) et/ou dans des articles sanitaires absorbants finis à la sortie de la ligne de production (1), comprenant les étapes suivantes :
- la détection des valeurs actuelles de paramètres de fonctionnement qui sont indicatifs du fonctionnement actuel d'au moins un dispositif de production (38, 40, 41, 42, 43) adapté pour effectuer des opérations de production respectives sur les articles sanitaires absorbants (2) en cours de traitement le long de la ligne de production (1) ;
- l'exécution d'une comparaison entre les valeurs actuelles des paramètres de fonctionnement détectés et les valeurs de référence prédéterminées respectives ;
- en présence, parmi lesdits paramètres de fonctionnement, d'au moins un paramètre de fonctionnement anormal dont la valeur actuelle n'est pas conforme aux valeurs de référence prédéterminées respectives, l'identification, parmi une pluralité de causes de dysfonctionnement possibles de la ligne (1), d'au moins une cause de dysfonctionnement actuelle responsable dudit au moins un paramètre de fonctionnement anormal ;
ladite au moins une cause actuelle de dysfonctionnement étant identifiée à partir dudit au moins un paramètre de fonctionnement anormal au moyen d'algorithmes de décision basés sur au moins l'une des informations suivantes : des données connues et/ou auto-apprises relatives aux corrélations entre ledit au moins un paramètre de fonctionnement anormal et ladite pluralité de causes possibles de dysfonctionnement de la ligne ; des informations actuelles et/ou historiques fournies manuellement par un opérateur en association avec ledit au moins un paramètre de fonctionnement anormal et/ou ladite pluralité de causes possibles de dysfonctionnement de la ligne ; des règles logiques connues et/ou auto-apprises ; et
au moyen desdits algorithmes de décision, une prédiction des défauts étant effectuée à partir de ladite au moins une cause actuelle de dysfonctionnement identifiée et sur la base d'au moins une des informations suivantes : des données connues et/ou auto-apprises relatives aux corrélations entre ladite au moins une cause actuelle de dysfonctionnement identifiée et au moins un défaut possible connu ; des informations actuelles et/ou historiques fournies manuellement par un opérateur en association avec ladite au moins une cause actuelle de dysfonctionnement identifiée et/ou avec ledit au moins un défaut possible connu ; des règles logiques connues et/ou auto-apprises.

2. Procédé selon la revendication 1, comprenant en outre une étape de stockage de ladite au moins une cause de dysfonctionnement actuelle identifiée en association avec ledit au moins un paramètre de fonctionnement anormal de manière à enrichir les données auto-apprises relatives aux corrélations entre ledit au moins un paramètre de fonctionnement anormal et ladite pluralité de causes de dysfonctionnement possibles de la ligne (1).

3. Procédé selon l'une quelconque des revendications précédentes, comprenant une étape de stockage des défauts prédits afin d'enrichir les données auto-apprises relatives aux corrélations entre ladite au moins une cause de dysfonctionnement actuelle identifiée et ledit au moins un défaut possible connu.

4. Procédé selon l'une quelconque des revendications précédentes, comprenant une étape de définition, au moyen desdits algorithmes de décision, d'une action corrective à entreprendre pour remédier à ladite au moins une cause de dysfonctionnement actuelle identifiée et auxdits défauts prédits ; l'action corrective à entreprendre portant sur un ou plusieurs des dispositifs de production (38, 40, 41, 42, 43) et/ou sur un ou plusieurs des dispositifs accessoires de la ligne de production (1), qui sont impliqués dans ladite cause de dysfonctionnement actuelle identifiée.

5. Procédé selon la revendication 4, comprenant en outre une étape de conversion automatique de l'action corrective à entreprendre en un signal de commande pour un ou plusieurs actionneurs (46) qui sont associés de manière fonctionnelle à un ou plusieurs dispositifs de production (38, 40, 41, 42, 43) et/ou à un ou plusieurs dispositifs accessoires qui sont impliqués dans la cause de dysfonctionnement actuelle identifiée.

6. Procédé selon l'une quelconque des revendications précédentes, comprenant une étape d'acquisition des images des articles sanitaires absorbants (2) en cours de traitement le long de la ligne de production (1) et/ou des articles sanitaires absorbants (2) en sortie de la ligne de production (1).

7. Procédé selon la revendication 6, comprenant une étape de détection de défauts supplémentaires par le traitement desdites images acquises, en plus de ladite prédiction des défauts.

8. Procédé selon l'une quelconque des revendications précédentes, les paramètres de fonctionnement détectés comprenant au moins l'un des éléments suivants : température ; pression ; vibration ; position des objets ; poids ; quantité ; niveau de vide ; état des jets d'air ; niveau d'énergie d'impact d'un dispositif de coupe (43) de la ligne (1) ; niveau de propreté ; présence ou quantité de matériau à une position prédéterminée ; tension d'alimentation des éléments des articles hygiéniques absorbants (2) en cours de traitement ; vitesse ; couple ; courant.

9. Procédé selon l'une quelconque des revendications précédentes, les défauts prédits au moyen desdits algorithmes de décision concernant au moins l'un des éléments suivants : étanchéité d'un joint, étanchéité d'un collage, répartition ou quantité de matière et degré d'absorption.

10. Procédé selon l'une quelconque des revendications précédentes, comprenant également une étape d'acquisition de données d'articles directement liées aux articles sanitaires absorbants en cours de traitement le long de la ligne de production et/ou aux articles sanitaires absorbants finis, en sortie de ligne.

11. Procédé selon la revendication 10, les données d'article concernant au moins l'un des éléments suivants : l'apparence, la forme, le poids, le positionnement, les dimensions et les contours des articles en cours de traitement le long de la ligne de production et/ou des articles sanitaires absorbants finis, à la sortie de la ligne.

12. Procédé selon la revendication 10 ou 11, comprenant une étape de détection de tout défaut supplémentaire par le traitement desdites données d'article, en plus de ladite prédiction de tout défaut.

13. Ligne de production (1) d'articles sanitaires absorbants (12) comprenant :
- des dispositifs de production (38, 40, 41, 42, 43) configurés pour effectuer des opérations de production respectives sur les articles sanitaires absorbants (2) en cours de traitement le long de la ligne de production (1) ;
- des capteurs (45) adaptés pour détecter les valeurs actuelles des paramètres de fonctionnement qui sont indicatifs du fonctionnement actuel d'au moins un des dispositifs de production (38, 40, 41, 42, 43) ;
- une unité de commande (60) comprenant une mémoire (61) et un processeur (62) configuré pour exécuter les étapes du procédé selon l'une quelconque des revendications 1 à 12.

14. Ligne de production (1) selon la revendication 13, les dispositifs de production (38, 40, 41, 42, 43) comprenant au moins l'un des éléments suivants :
- des organes de transport (38) adaptés pour supporter et déplacer les articles sanitaires absorbants (2) en cours de traitement le long de la ligne (1) ;
- des dispositifs d'alimentation (40) adaptés pour alimenter des éléments des articles sanitaires absorbants (2) en cours de traitement et pour les placer, au moins partiellement en chevauchement mutuel, sur une surface de support (47) des organes de transport (38) ;
- des éléments de retenue (41) configurés pour retenir en position les éléments placés sur la surface de support (47) des organes de transport (38), comprenant de préférence des dispositifs d'aspiration actifs sur des trous de retenue formés sur la surface de support (47) des organes de transport (38) ;
- au moins un dispositif de fixation (42) adapté pour fixer les éléments des articles sanitaires absorbants (2) étant traités ensemble ;
- au moins un dispositif de coupe (43) adapté pour couper des éléments des articles sanitaires absorbants (2) en cours de traitement et/ou une bande continue (44) d'articles sanitaires absorbants (2) en cours de traitement assemblés, résultant du processus de production, en articles individuels (2) ;

15. Ligne de production (1) selon l'une quelconque des revendications 13 et 14, les capteurs (45) comprenant au moins l'un des capteurs suivants : capteur de température ; capteur de pression ; capteur de vibration ; capteur de position ; capteur de poids ; capteur de quantité ; capteur de débit ; capteur de niveau de vide ; capteur d'état de jet d'air ; capteur optique ; capteur de tension ; capteur de vitesse ; capteur de couple ; capteur de courant.
